# EUROPÄISCHE PATENTSCHRIFT

(11) **EP 4 574 890 B1**
(45) Veröffentlichungstag und Bekanntmachung des Hinweises auf die Patenterteilung: **22.10.2025**
(21) Anmeldenummer: 23220150.9
(22) Anmeldetag: 22.12.2023
(51) Int. Cl.: C08K 5/103, C08L 67/04

(54) **SPRITZGEGOSSENES BAUTEIL, VERFAHREN ZU DESSEN HERSTELLUNG UND VERWENDUNG DES SPRITZGEGOSSENEN BAUTEILS**
INJECTION MOULDED COMPONENT, METHOD FOR THE PRODUCTION THEREOF AND USE OF THE INJECTION MOULDED COMPONENT
COMPOSANT MOULÉ PAR INJECTION, SON PROCÉDÉ DE FABRICATION ET UTILISATION DU COMPOSANT MOULÉ PAR INJECTION

(43) Veröffentlichungstag der Anmeldung: 25.06.2025
(73) Patentinhaber: Green Elephant Biotech GmbH, 35394 Gießen (DE); Hille, Johannes, Singapur 437918 (SG)
(72) Erfinder: HILLE, Johannes, Singapur 437918 (SG); KÄSSER, Lukas, 35460 Staufenberg (DE); MÜLLER, Fabian, 57462 Olpe (DE)
(74) Vertreter: Metten, Karl-Heinz

(56) Entgegenhaltungen:
- JP-A- 2015 105 284
- JP-A- H1 036 650
- JP-A- H1 121 438
- US-A1- 2012 009 103

## Beschreibung

Die vorliegende Erfindung betrifft ein spritzgegossenes Bauteil enthaltend oder gebildet aus einem pflanzenbasierten und/oder, insbesondere und, biologisch abbaubaren thermoplastischen Polymer, insbesondere Polylactiden. Ferner betrifft die Erfindung ein Verfahren zur Herstellung des erfindungsgemäßen Bauteils sowie die Verwendung dieses Bauteils.

Polylactide, auch Polymilchsäure genannt, finden als Ersatz für herkömmliche, letztendlich auf Erdöl zurückgehende Thermoplaste ein wachsendes Interesse. Polylactide können nachhaltig gewonnen werden, beispielsweise aus nachwachsenden Rohstoffen wie Reis, Weizen, Mais, Kartoffelstärke und Melasse. Bislang werden Polylactide häufig im Gemisch mit herkömmlichen Thermoplasten wie Polyethylenterephthalat oder Polybutylenterephthalat eingesetzt.

Beispielsweise werden in der DE 197 55 966 A1 Blends aus Polyhydroxybutyraten und Polylactiden beschrieben, welche zu spritzgegossenen Bauteilen führen, die sich durch eine gute Protein-Affinität auszeichnen sollen. Als weitere Komponenten kommen hierbei Weichmacher und Nukleierungsmittel zum Einsatz.

Die EP 3 875 572 A1 befasst sich mit Zellkulturbehältern, die aus einer Mischung aus Polylactidpolymeren gebildet sind, wobei diese Mischungen (100 - x) Gew.-% Poly-L-Lactid und x-Gew.-% Poly-D-Lactid aufzuweisen haben und wobei x ≤ 25 zu sein hat. Die in der EP 3 875 572 A1 beschriebenen Zellkulturbehälter sollen auch ohne zusätzliche Behandlung oder Beschichtung bei der Kultivierung verankerungsabhängiger tierischer oder pflanzlicher Zellen dennoch eine gute Zellanhaftung und eine robuste Zellverankerung gewährleisten.

Die CN 107 108 901 A betrifft ein Verfahren zur Herstellung von geformten Polymilchsäurenprodukten, enthaltend stereokomplexe Polymilchsäuren (sc-PLA). Gemäß diesem Verfahren hat man ein erstes Milchsäurehomopolymer mit einem Überschuss eines zweiten Milchsäurehomopolymers im geschmolzenen Zustand zu vermischen, wobei die ersten und zweiten Milchsäurehomopolymere ausgewählt sind aus Poly-D-Milchsäure-Homopolymer und Poly-L-Milchsäure-Homopolymer. Die hierbei erhaltene gehärtete Mischung wird im festen Zustand mit einer weiteren Menge an dem ersten Milchsäurehomopolymer, ebenfalls im festen Zustand, vermischt. Anschließend hat eine Schmelzeverarbeitung dieses Gemisches bei einer Temperatur stattzufinden, die höher ist als der Schmelzpunkt des Poly-D- und des Poly-L-Milchsäurehomopolymers und die niedriger ist als der Schmelzpunkt von sc-PLA. Nach Abkühlen des schmelzevermengten Produktes erhält man einen geformten Polymilchsäureartikel.

Die CN 102 206 406 A betrifft ein Verfahren zur Herstellung eines transparenten, hitzebeständigen modifizierten Polymilchsäurematerials. Das Herstellungsverfahren bedient sich dabei gleichzeitig der Anwesenheit eines Keimbildners zur Änderung des Kristallisationszustandes der Polymilchsäure sowie eines Kettenverlängerers zur Vernetzung und damit zur Änderung der Molekularstruktur der Polymilchsäure. Das transparente, hitzebeständige Polymilchsäureprodukt hat neben der vorangehend beschriebenen modifizierten Polymilchsäure ein Chitin-Whisker-Polymethylmethacrylat, einen Kettenverlängerer, oligomere Polymilchsäure und Tris(nonylphenyl)phosphit zu enthalten.

Die JP H11 21438 A betrifft eine eingefärbte Zusammensetzung enthaltend ein Pigment und einen mehrwertigen Alkohol-Fettsäureester als Pigmentdispergiermittel sowie Polymilchsäure oder ein Polymilchsäure/aliphatisches Polyester-Copolymer. Diese Zusammensetzung soll sich durch eine gute Dispergierbarkeit des Pigments auszeichnen.

Die JP 2015/105284 A stellt ab auf ein Verfahren zur Herstellung einer Polymilchsäureharz-Zusammensetzung umfassend das Mischen eines Pigments und eines Dispergiermittels, ausgewählt aus der Gruppe bestehend aus Fettsäureamiden mit 8 bis 31 Kohlenstoffatomen und Fettsäureestern mit 8 bis 31 Kohlenstoffatomen, sowie das Schmelzkneten der erhaltenen Pigmentmischung mit einer Komponente, enthaltend ein Polymilchsäureharz, ein Flammschutzmittel und eine Carbodiimidverbindung. Aus der Polymilchsäureharz-Zusammensetzung erhältliche Formteile sollen sich durch eine gute Flammhemmung, Schlagfestigkeit und Haltbarkeit auszeichnen.

Aus der JP H10 36650 A geht eine biologisch abbaubare Polymerzusammensetzung auf Polymilchsäurebasis enthaltend ein nichtionisches Antistatikmittel in Form eines Glycerinfettsäureesters hervor, die über gute antistatische Eigenschaften und Transparenz verfügen soll.

Die nach dem Stand der Technik erhältlichen spritzgegossenen Produkte auf Basis von Polylactiden lassen noch stets Wünsche offen, beispielsweise hinsichtlich des Auftretens von Verzug beim Abkühlungsprozess. Auch werden beim Spritzgießprozess Scherungsphänomene beobachtet, welche sich als abträglich für die optischen und mechanischen Eigenschaften des fertigen Bauteils erwiesen haben.

Der vorliegenden Erfindung hat daher die Aufgabe zugrunde gelegen, spritzgegossene Bauteile verfügbar zu machen, die nicht mit den Nachteilen des Stands der Technik behaftet sind und die insbesondere ein nachhaltiges, ressourcenschonendes Wirtschaften ermöglichen, ohne Einbußen bei den mechanischen und/oder optischen Eigenschaften in Kauf nehmen zu müssen.

Demgemäß wurde ein spritzgegossenes Bauteil gefunden, enthaltend oder gebildet aus einem pflanzenbasierten und/oder, insbesondere und, biologisch abbaubaren thermoplastischen Polymer, insbesondere Polylactiden, und mindestens einem Monoglycerid einer Fettsäure, insbesondere einer natürlichen Fettsäure, wobei der Gehalt an dem pflanzenbasierten und/oder biologisch abbaubaren thermoplastischen Polymer, insbesondere an Polylactiden, jeweils bezogen auf das Gesamtgewicht des Bauteils, mindestens 95,0 Gew.-%, bevorzugt mindestens 96,0 Gew.-% und besonders bevorzugt mindestens 97,0 Gew.-%, beträgt und wobei das spritzgegossene Bauteil mindestens zwei Anspritzpunkte, insbesondere mindestens drei Anspritzpunkte, aufweist. Bevorzugt kommen hierbei pflanzenbasierte und biologisch abbaubare thermoplastische Polymere zum Einsatz. Ein Bauteil im Sinne der vorliegenden Erfindung kann auch als Formkörper bezeichnet werden.

Die der Erfindung zugrunde liegende Aufgabe wird demgemäß bevorzugt gelöst durch ein spritzgegossenes Bauteil, enthaltend oder gebildet aus Polylactiden und mindestens einem Monoglycerid einer Fettsäure, insbesondere einer natürlichen Fettsäure, wobei der Gehalt an Polylactiden, jeweils bezogen auf das Gesamtgewicht des Bauteils, mindestens 95,0 Gew.-%, bevorzugt mindestens 96,0 Gew.-% und besonders bevorzugt mindestens 97,0 Gew.-%, beträgt.

Geeignete pflanzenbasierte und biologisch abbaubare thermoplastische Polymere umfassen insbesondere Polylactide, Polyhydroxyalkanoate, Poly(butylensuccinate) und deren beliebigen Mischungen. Als Polyhydroxyalkanoate kommen insbesondere Polyhydroxybutyrat, Polyhydroxyvalerat und Polyhydroxybutyrat-co-hydroxyvaleriansäure in Betracht. Unter den pflanzenbasierten und biologisch abbaubaren thermoplastischen Polymeren wird besonders bevorzugt auf Polylactide zurückgegriffen.

In einer bevorzugten Ausführungsform besteht das erfindungsgemäße spritzgegossene Bauteil aus mindestens einem Monoglycerid einer Fettsäure, insbesondere einer natürlichen Fettsäure, und mindestens einem pflanzenbasierten und/oder, insbesondere und, biologisch abbaubaren thermoplastischen Polymer. In einer besonders bevorzugten Ausführungsform besteht das erfindungsgemäße spritzgegossene Bauteil aus mindestens einem Monoglycerid einer Fettsäure, insbesondere einer natürlichen Fettsäure, und Polylactiden. Bei Polylactiden im Sinne der vorliegenden Erfindung kann es sich sowohl um eine Mischung aus unterschiedlichen Polylactiden, beispielsweise um eine Mischung aus Polylactiden mit unterschiedlichen Molekulargewichten und/oder unterschiedlichen stereochemischen Zusammensetzungen, handeln als auch um ein einheitliches Polylactid-Homopolymer.

Polylactide zählen zu den Polyestern. Sie sind aus chemisch miteinander verbundenen Milchsäurewiederholungseinheiten aufgebaut. Polylactide können durch direkte Kondensationsreaktionen von Milchsäuremolekülen wie auch über ionische Polymerisation von Lactid erhalten werden. Lactid stellt die dimere Form der Milchsäure dar und liegt demgemäß als Sechsring vor. Das Lactid ist z.B. durch Vergärung von Melasse oder durch Fermentation von Glucose zugänglich. Milchsäure wird in der Regel ebenfalls fermentativ über geeignete Milchsäurebakterien gewonnen. Die bei der Fermentation zum Einsatz kommenden Kohlenhydrate stellen im Allgemeinen Hexosen oder Verbindungen dar, die leicht in Hexosen umgewandelt werden können. Sie können gewonnen werden bzw. liegen z.B. vor in Reis, Weizen, Mais, Kartoffelstärke und Melasse. Milchsäureproduzierende Mikroorganismen stellen z.B. Lactobazillus- und Rhizopus-Stämme dar, wobei für die industrielle Produktion regelmäßig grampositive, nicht sporenbildende Lactobazillen verwendet werden. Während man mit homofermentativen Lactobazillen stereospezifisch L(+)-Milchsäure erhält, liefern heterofermentative Lactobazillen regelmäßig ein Gemisch aus L- und D-Milchsäure. Die fermentative Herstellung liefert einen effizienten Zugang zu den reinen Enantiomeren der Milchsäure. Diese reinen Milchsäureenantiomere können mit Hilfe des Enzyms Lactat-Racemase auch in das Racemat überführt werden. Besonders bevorzugt liegen in den erfindungsgemäßen spritzgegossenen Bauteilen überwiegend sowie insbesondere im Wesentlichen ausschließlich, vorzugsweise ausschließlich, L-Milchsäureeinheiten vor.

Geeignete Fettsäuren als Bestandteil der Komponente Monoglycerid einer Fettsäure umfassen verzweigte und geradkettige Fettsäuren, z.B. verzweigte und/oder geradkettige C4- bis C32-Fettsäuren, insbesondere C8- bis C24-Fettsäuren. Die Fettsäuren können gesättigte oder ungesättigte Fettsäuren darstellen. Exemplarisch seien Stearinsäure, Palmitinsäure und Ölsäure sowie deren beliebige Mischungen genannt. Auch kommen z.B. Decylsäure und 2-Ethylhexansäure in Betracht. Grundsätzlich sind natürliche Fettsäuren bevorzugt. Natürliche Fettsäuren besitzen in der Regel eine gerade Zahl von Kohlenstoffatomen, z.B. C4 bis C32 und insbesondere C8 bis C24, und sind unverzweigt. Bevorzugt wird auf Fettsäuren pflanzlichen Ursprungs zurückgegriffen.

Derartige erfindungsgemäße spritzgegossene Bauteile lösen die der Erfindung zugrunde liegende Aufgabenstellung besonders zufriedenstellend, die über einen Gehalt an dem mindestens einen Monoglycerid einer Fettsäure von maximal 5,0 Gew.-%, bevorzugt von maximal 2,5 Gew.-% und besonders bevorzugt im Bereich von 0,5 bis 2,0 Gew.-%, jeweils bezogen auf das Gesamtgewicht des Bauteils, verfügen. Hierbei kommen insbesondere auch solche erfindungsgemäßen spritzgegossenen Bauteile zum Einsatz mit einem einen Gehalt an dem mindestens einen Monoglycerid einer Fettsäure, insbesondere einer natürlichen Fettsäure, im Bereich von 0,5 bis 2,5 Gew.-%, bevorzugt im Bereich von 0,75 bis 2,25 Gew.-% und besonders bevorzugt im Bereich von 1,0 bis 2,0 Gew.-%, jeweils bezogen auf das Gesamtgewicht des Bauteils.

Dabei kann für bevorzugte erfindungsgemäße spritzgegossene Bauteile, die mindestens ein Monoglycerid einer Fettsäure und ein pflanzenbasiertes und/oder, insbesondere und, biologisch abbaubares thermoplastisches Polymer, vorzugsweise Polylactide, enthalten und die vorzugsweise aus mindestens einem Monoglycerid einer Fettsäure und dem pflanzenbasierten und/oder, insbesondere und, biologisch abbaubaren thermoplastischen Polymer, vorzugsweise Polylactide, bestehen, vorgesehen sein, dass der Gehalt an dem pflanzenbasierten und/oder, insbesondere und, biologisch abbaubaren thermoplastischen Polymer, vorzugsweise Polylactide, im Bereich von 95,0 bis 99,5 Gew.-% und der Gehalt an dem mindestens einen Monoglycerid im Bereich von 0,5 bis 5,0 Gew.-% liegt, wobei bevorzugt der Gehalt an dem pflanzenbasierten und/oder, insbesondere und, biologisch abbaubaren thermoplastischen Polymer, vorzugsweise Polylactide, im Bereich von 96,0 bis 99,0 Gew.-% und der Gehalt an dem mindestens einen Monoglycerid im Bereich von 1,0 bis 4,0 Gew.-% liegt und wobei besonders bevorzugt der Gehalt an dem pflanzenbasierten und/oder, insbesondere und, biologisch abbaubaren thermoplastischen Polymer, vorzugsweise Polylactide, im Bereich von 97,0 bis 98,5 Gew.-% und der Gehalt an dem mindestens einen Monoglycerid im Bereich von 1,5 bis 3,0 Gew.-% liegt, wobei die Anteile der das erfindungsgemäße spritzgegossene Bauteil bildenden Komponenten jeweils stets 100,0 Gew.-% ergeben.

In weiteren zweckmäßigen Ausführungsformen der erfindungsgemäßen spritzgegossenen Bauteile können in diesen auch freie Fettsäuren, insbesondere freie natürliche Fettsäuren, vorliegen. Der Gehalt an freien Fettsäuren, insbesondere an freien natürlichen Fettsäuren, ist in diesen spritzgegossenen Bauteilen vorzugsweise kleiner 0,5 Gew.-%, bevorzugt kleiner 0,3 Gew.-% und besonders bevorzugt kleiner 0,1 Gew.-%, jeweils bezogen auf das Gesamtgewicht des Bauteils.

Dabei kann für bevorzugte erfindungsgemäße spritzgegossene Bauteile, die mindestens ein Monoglycerid einer Fettsäure, mindestens eine freie Fettsäure, insbesondere eine freie natürliche Fettsäure, und ein pflanzenbasiertes und/oder, insbesondere und, biologisch abbaubares thermoplastisches Polymer, vorzugsweise Polylactide, enthalten und die vorzugsweise aus mindestens einem Monoglycerid einer Fettsäure, mindestens einer freien Fettsäure und dem pflanzenbasierten und/oder, insbesondere und, biologisch abbaubaren thermoplastischen Polymer, vorzugsweise Polylactide, bestehen, vorgesehen sein, dass der Gehalt an dem pflanzenbasierten und/oder, insbesondere und, biologisch abbaubaren thermoplastischen Polymer, vorzugsweise Polylactide, im Bereich von 95,0 bis 99,5 Gew.-%, der Gehalt an dem mindestens einen Monoglycerid im Bereich von 0,45 bis 4,5 Gew.-% und der Gehalt an der mindestens einen freien Fettsäure im Bereich von 0,05 bis 0,5 Gew.-% liegt, wobei bevorzugt der Gehalt an dem pflanzenbasierten und/oder, insbesondere und, biologisch abbaubaren thermoplastischen Polymer, vorzugsweise Polylactide, im Bereich von 96,0 bis 99,0 Gew.-%, der Gehalt an dem mindestens einen Monoglycerid im Bereich von 0,93 bis 3,7 Gew.-% und der Gehalt an der mindestens einen freien Fettsäure im Bereich von 0,07 bis 0,3 Gew.-% liegt und wobei besonders bevorzugt der Gehalt an dem pflanzenbasierten und/oder, insbesondere und, biologisch abbaubaren thermoplastischen Polymer, vorzugsweise Polylactide, im Bereich von 97,0 bis 98,5 Gew.-%, der Gehalt an dem mindestens einen Monoglycerid im Bereich von 1,41 bis 2,8 Gew.-% und der Gehalt an der mindestens einen freien Fettsäure im Bereich von 0,09 bis 0,2 Gew.-% liegt, wobei die Anteile der das erfindungsgemäße spritzgegossene Bauteil bildenden Komponenten jeweils stets 100,0 Gew.-% ergeben.

In weiteren zweckmäßigen Ausführungsformen der erfindungsgemäßen spritzgegossenen Bauteile kann in diesen auch Glycerin vorliegen. Der Gehalt an Glycerin ist in diesen spritzgegossenen Bauteilen vorzugsweise kleiner 0,3 Gew.-%, bevorzugt kleiner 0,15 Gew.-% und besonders bevorzugt kleiner 0,075 Gew.-%, jeweils bezogen auf das Gesamtgewicht des Bauteils.

Dabei kann für bevorzugte erfindungsgemäße spritzgegossene Bauteile, die mindestens ein Monoglycerid einer Fettsäure, mindestens eine freie Fettsäure, insbesondere eine freie natürliche Fettsäure, Glycerin und ein pflanzenbasiertes und/oder, insbesondere und, biologisch abbaubares thermoplastisches Polymer, vorzugsweise Polylactide, enthalten und die vorzugsweise aus mindestens einem Monoglycerid einer Fettsäure, mindestens einer freien Fettsäure, Glycerin und dem pflanzenbasierten und/oder, insbesondere und, biologisch abbaubaren thermoplastischen Polymer, vorzugsweise Polylactide, bestehen, vorgesehen sein, dass der Gehalt an dem pflanzenbasierten und/oder, insbesondere und, biologisch abbaubaren thermoplastischen Polymer, vorzugsweise Polylactide, im Bereich von 95,0 bis 99,5 Gew.-%, der Gehalt an dem mindestens einen Monoglycerid im Bereich von 0,45 bis 4,2 Gew.-%, der Gehalt an der mindestens einen freien Fettsäure im Bereich von 0,04 bis 0,4 Gew.-% und der Gehalt an Glycerin im Bereich von 0,01 bis 0,4 Gew.-% liegt, wobei bevorzugt der Gehalt an dem pflanzenbasierten und/oder, insbesondere und, biologisch abbaubaren thermoplastischen Polymer, vorzugsweise Polylactide, im Bereich von 96,0 bis 99,0 Gew.-%, der Gehalt an dem mindestens einen Monoglycerid im Bereich von 0,92 bis 3,4 Gew.-%, der Gehalt an der mindestens einen freien Fettsäure im Bereich von 0,06 bis 0,3 Gew.-% und der Gehalt an Glycerin im Bereich von 0,02 bis 0,3 Gew.-% liegt und wobei besonders bevorzugt der Gehalt an dem pflanzenbasierten und/oder, insbesondere und, biologisch abbaubaren thermoplastischen Polymer, vorzugsweise Polylactide, im Bereich von 97,0 bis 98,5 Gew.-%, der Gehalt an dem mindestens einen Monoglycerid im Bereich von 1,39 bis 2,7 Gew.-%, der Gehalt an der mindestens einen freien Fettsäure im Bereich von 0,08 bis 0,2 Gew.-% und der Gehalt an Glycerin im Bereich von 0,03 bis 0,1 Gew.-% liegt, wobei die Anteile der das erfindungsgemäße spritzgegossene Bauteil bildenden Komponenten jeweils stets 100,0 Gew.-% ergeben.

Besonders geeignete erfindungsgemäße spritzgegossene Bauteile, vorzugsweise auf Basis von Polylactiden, insbesondere wenn in Form eine Wellplatte vorliegend, zeichnen sich auch dadurch aus, dass sie im Bereich von 250 bis 290 nm über eine kleinere, bevorzugt über eine um mindestens 75 % kleinere und besonders bevorzugt über eine um mindestens 50 % kleinere Absorption verfügen als ein korrespondierendes spritzgegossenes Bauteil, gebildet aus Polystyrol. Bevorzugt sind auch solche erfindungsgemäßen spritzgegossenen Bauteile, insbesondere wenn in Form eine Wellplatte vorliegend, die eine Fluoreszenz zeigen, welche bei Anregung mit einer Wellenlänge von 250 nm und/oder 450 nm und/oder 500 nm vergleichbar ist mit der eines korrespondierenden spritzgegossenen Bauteils, gebildet aus Polystyrol.

Erfindungsgemäße spritzgegossene Bauteile können auch über mindestens ein Pigment oder mindestens einen Farbstoff verfügen. Diese Pigmente oder Farbstoffe können als Bestandteil des Polylactids, als Bestandteil des Monoglycerids und/oder als separate Komponente bei der Herstellung der erfindungsgemäßen spritzgegossenen Bauteile in diese eingearbeitet werden. Vielfach hat es sich als besonders zweckmäßig erwiesen, das Pigment oder den Farbstoff als Bestandteil eines Masterbatches, vorzugsweise auf Basis des für das spritzgegossene Bauteil verwendeten pflanzenbasierten und/oder, insbesondere und, biologisch abbaubaren thermoplastischen Polymers, beispielsweise in Form des Polylactids, mit diesem pflanzenbasierten und/oder, insbesondere und, biologisch abbaubaren thermoplastischen Polymer zu vermengen, insbesondere vor dem Aufschmelzvorgang, oder in das schmelzflüssige pflanzenbasierte und/oder, insbesondere und, biologisch abbaubare thermoplastische Polymer, vorzugsweise Polylactide, einzuarbeiten. Bevorzugt kommen hierbei pflanzenbasierte und/oder biologisch abbaubare Pigmente oder Farbstoffe, besonders bevorzugt pflanzenbasierte und biologisch abbaubare Pigmente oder Farbstoffe zum Einsatz.

Die pflanzenbasierten und/oder, insbesondere und, biologisch abbaubaren thermoplastischen Polymere, vorzugsweise die Polylactide, werden bei der Herstellung der erfindungsgemäßen Bauteile mittels Spritzgießens im Allgemeinen in Granulatform eingesetzt. Der Wassergehalt der, insbesondere in Granulatform vorliegenden, Polylactide liegt vorzugsweise nicht oberhalb von 800 ppm, bevorzugt nicht oberhalb von 600 ppm und besonders bevorzugt nicht oberhalb von 500 ppm.

Die erfindungsgemäßen spritzgegossenen Bauteile können in transparenter wie auch in transluzenter Ausgestaltung erhalten werden.

Die der Erfindung zugrunde liegende Aufgabe wird auch gelöst durch ein Verfahren zur Herstellung eines erfindungsgemäßen spritzgegossenen Bauteils, bei dem man das pflanzenbasierte und/oder, insbesondere und, biologisch abbaubare thermoplastische Polymer, insbesondere Polylactide, in schmelzflüssiger Form über mindestens zwei, insbesondere mindestens drei Angusskanäle in die Spritzgießform für das Bauteil einführt.

Überraschend hat sich gezeigt, dass besonders zufriedenstellende optische und mechanische Eigenschaften mit solchen erfindungsgemäßen spritzgegossenen Bauteilen erhalten werden, die mindestens zwei Anspritzpunkte, insbesondere mindestens drei Anspritzpunkte, aufweisen, d.h. bei denen das schmelzeflüssige Produkt über mindestens zwei, bevorzugt mindestens drei Angusskanäle in die Gussform eingeführt wurde.

Das erfindungsgemäße spritzgegossene Bauteil lässt sich unproblematisch aus der Spritzgießform entnehmen, und zwar auch bereits in einem frühen Stadium nach dem Spritzgießvorgang, d.h. im noch warmen Zustand. Das erfindungsgemäße Bauteil kann demgemäß auch außerhalb der Spritzgießform abgekühlt werden, womit relativ kurze Zykluszeiten einhergehen.

Bei dem erfindungsgemäßen Verfahren vermengt man das pflanzenbasierte und/oder, insbesondere und, biologisch abbaubare thermoplastische Polymer bzw. besonders bevorzugt Polylactide in Pulver- oder Granulatform mit dem mindestens einem Monoglycerid einer Fettsäure, insbesondere einer natürlichen Fettsäure, vorzugsweise vor dem Aufschmelzen des pflanzenbasierten und/oder, insbesondere und, biologisch abbaubaren thermoplastischen Polymers, insbesondere Polylactide. Besonders einwandfreie erfindungsgemäße spritzgegossene Bauteile werden bei Verwendung von Polylactiden mit einem Wassergehalt von nicht mehr als 800 ppm, bevorzugt von nicht mehr als 600 ppm und besonders bevorzugt von nicht mehr als 500 ppm sowie insbesondere von nicht mehr als 300 ppm erhalten. Erfindungsgemäße spritzgegossene Bauteile können z.B. ausgewählt sein aus der Gruppe bestehend aus Well-Platten (auch Mikrotiterplatten genannt), Well-Plattendeckeln, Teststäbchen, Teströhrchen, Testkugeln, Petrischalen, Kulturmedienbehältern, insbesondere Kulturmedienflaschen, Pipetten, insbesondere serologischen Pipetten, Pipettenbauteilen, Implantaten, Handyhüllen, Behältnissen für Lebensmittel oder Kosmetika, Kolben, insbesondere Erlenmeyer-Kolben, Zellkulturgefäßen, insbesondere Schüttelkolben wie Schüttelkolben mit Schikanen, T-Flaschen, Reaktionsgefäßen, Schraubdeckeln, Elektrophoresekammern, Halterungen für Reaktionsgefäße, Probenröhrchen, insbesondere Stuhlprobenröhrchen, Blutprobenröhrchen oder Abstrichprobenröhrchen, Halterungen für Probenröhrchen, Urinprobenbechern, Halterungen für Urinprobenbecher, Gehäuse für Antigen-Teststreifen, Lab-on-Chip Systeme, Organ-on-Chip Systeme, Küvetten, Reagenzreservoire, Mikroträger, Haltevorrichtungen zum Transport von medizinischen Behältern, insbesondere Spritzennester, Spritzentuben, Zellzählkammern, Filtergehäusen, Gehäusen für Bioreaktoren, Bioreaktorkesseln, Pipettenspitzen-Boxen, Abwurfbehältern, insbesondere für Spritzenkanülen und/oder Glas, Schutzkappen für Spritzenkanülen, Spritzen oder Spritzenstempeln. Besonders zufriedenstellende Ergebnisse stellen sich auch mit Well-Platten als erfindungsgemäße spritzgegossene Bauteile ein.

Mit der vorliegenden Erfindung geht die überraschende Erkenntnis einher, dass erfindungsgemäße spritzgegossene Bauteile aus nachwachsenden Rohstoffen, insbesondere Polylactiden, zugänglich sind, die nach erfolgter Verwendung bzw. Verwertung einem ressourcenschonenden Recycling zugänglich sind bzw. biologisch in die Ausgangsverbindungen zerlegt werden können. Auch zeichnen sich die erfindungsgemäßen spritzgegossenen Bauteile, insbesondere bei Verwendung von Polylactiden, durch geringen Verzug beim Abkühlen aus. Überdies findet bei der Einleitung des schmelzflüssigen Materials, insbesondere auf Basis von Polylactiden, in die Spritzgussform, insbesondere wenn über zwei oder mehr Angusskanäle eingeführt, keine bzw. keine ausgeprägte Scherung statt. Die erfindungsgemäßen spritzgegossenen Bauteile, insbesondere auf Basis von Polylactiden, zeichnen sich demgemäß auch dadurch aus, dass sie klar und schlierenfrei sind. Die erfindungsgemäßen Bauteile, insbesondere auf Basis von Polylactiden, stehen daher besonders bevorzugt auch für den Einsatz im Zusammenhang mit optischen Analyseverfahren zur Verfügung, z.B. um darin zu vermessende Proben aufzunehmen. Demgemäß handelt es sich bei besonders bevorzugten erfindungsgemäßen spritzgegossenen Bauteilen, insbesondere auf Basis von Polylactiden, um sogenannte Well-Platten.

Insbesondere hat sich auch überraschend gezeigt, dass die erfindungsgemäßen spritzgegossenen Bauteile, insbesondere auf Basis von Polylactiden, gegenüber korrespondierenden Bauteilen aus Polystyrol über vorteilhafte Eigenschaften verfügen, beispielsweise bei 250 nm eine signifikant geringere Absorption aufweisen. Auch hat sich überraschend gezeigt, dass die erfindungsgemäßen spritzgegossenen Bauteile, insbesondere auf Basis von Polylactiden, bei einer Anregungswellenlänge von 250 nm wie auch bei 300 nm eine deutlich geringere Eigenfluoreszenz als korrespondierende spritzgegossene Polystyrolbauteile aufweisen. Darüber hinaus hat sich gezeigt, dass die erfindungsgemäßen spritzgegossenen Bauteile, insbesondere auf Basis von Polylactiden, im Hinblick auf die Adhäsion von Proteinen wie auch von Doppelstrang-DNA (dsDNA) an der Oberfläche im Wesentlichen vergleichbar sind mit korrespondierenden spritzgegossenen Bauteilen aus Polystyrol. Beispielsweise konnten nach einem einmaligen Waschschritt mit PBS-Puffer (wässrige phosphathaltige Pufferlösung, enthaltend Natriumchlorid, Kaliumchlorid, Dinatriumhydrogenphosphat und Kaliumdihydrogenphosphat) regelmäßig weniger als 5 Gew.-% an BSA (Bovines Serumalbumin), bezogen auf die Gesamtmenge an auf dem erfindungsgemäßen spritzgegossenen Bauteil aufgebrachten BSA, mittels BCA-Tests (nach P. K. Smith et al., "Measurement of protein using bicinchoninic acid" in Analytical biochemistry. Band 150, Nr. 1, Oktober 1985, Seiten 76 bis 85) nachgewiesen werden. Auch lag nach einem einmaligen Waschschritt mit PBS-Puffer (wässrige phosphathaltige Pufferlösung, enthaltend Natriumchlorid, Kaliumchlorid, Dinatriumhydrogenphosphat und Kaliumdihydrogenphosphat) die Menge an Doppelstrang-DNA ermittelt mit dem PicoGreen-Assay regelmäßig unterhalb der Nachweisgrenze (*Limit of Detection*)*.* An den erfindungsgemäßen spritzgegossenen Bauteilen, insbesondere auf Basis von Polylactiden, findet demgemäß keine ausgeprägte Adhäsion von dsDNA statt.

Schließlich ist von Vorteil, dass sämtliche der die erfindungsgemäßen spritzgegossenen Bauteile bzw. Formkörper bildenden Komponenten wie Polylactide und Fettsäuren bzw. die Monoglyceride der Fettsäuren aus nachwachsenden Rohstoffen gewonnen werden können und damit ein sehr nachhaltiges Wirtschaften ermöglichen.

## Patentansprüche

1. Spritzgegossenes Bauteil, enthaltend oder gebildet aus
einem pflanzenbasierten und/oder, insbesondere und, biologisch abbaubaren thermoplastischen Polymer, insbesondere Polylactiden, und
mindestens einem Monoglycerid einer Fettsäure, insbesondere einer natürlichen Fettsäure,
**gekennzeichnet durch**
einen Gehalt an dem pflanzenbasierten und/oder, insbesondere und, biologisch abbaubaren thermoplastischen Polymer, insbesondere an Polylactiden, von mindestens 95,0 Gew.-%, bevorzugt von mindestens 96,0 Gew.-% und besonders bevorzugt von mindestens 97,0 Gew.-%, jeweils bezogen auf das Gesamtgewicht des Bauteils, und
mindestens zwei Anspritzpunkte.

2. Spritzgegossenes Bauteil nach Anspruch 1, **dadurch gekennzeichnet, dass** das pflanzenbasierte und/oder, insbesondere und, biologisch abbaubare thermoplastische Polymer ausgewählt ist aus der Gruppe bestehend aus Polylactiden, Polyhydroxyalkanoaten, Poly(butylensuccinaten) und deren beliebigen Mischungen, wobei Polylactide bevorzugt sind.

3. Spritzgegossenes Bauteil nach Anspruch 1 oder 2, **gekennzeichnet durch** mindestens drei Anspritzpunkte.

4. Spritzgegossenes Bauteil nach einem der vorangehenden Ansprüche,
**gekennzeichnet durch**
einen Gehalt an dem mindestens einen Monoglycerid einer Fettsäure, insbesondere einer natürlichen Fettsäure, von maximal 5,0 Gew.-%, bevorzugt von maximal 2,5 Gew.-% und besonders bevorzugt von maximal 2,0 Gew.-%, jeweils bezogen auf das Gesamtgewicht des Bauteils, oder **gekennzeichnet durch**
einen Gehalt an dem mindestens einen Monoglycerid einer Fettsäure, insbesondere einer natürlichen Fettsäure, im Bereich von 0,5 bis 2,5 Gew.-%, bevorzugt im Bereich von 0,75 bis 2,25 Gew.-% und besonders bevorzugt im Bereich von 1,0 bis 2,0 Gew.-%, jeweils bezogen auf das Gesamtgewicht des Bauteils.

5. Spritzgegossenes Bauteil nach einem der vorangehenden Ansprüche,
**gekennzeichnet durch**
einen Gehalt an freien Fettsäuren, insbesondere natürlichen Fettsäuren, kleiner 0,5 Gew.-%, bevorzugt kleiner 0,3 Gew.-% und besonders bevorzugt kleiner 0,1 Gew.-%, jeweils bezogen auf das Gesamtgewicht des Bauteils, und/oder, insbesondere und,
**gekennzeichnet durch** einen Gehalt an Glycerin kleiner 0,3 Gew.-%, bevorzugt kleiner 0,15 Gew.-% und besonders bevorzugt kleiner 0,075 Gew.-%, jeweils bezogen auf das Gesamtgewicht des Bauteils.

6. Spritzgegossenes Bauteil nach einem der vorangehenden Ansprüche, **dadurch**
**gekennzeichnet, dass**
dieses im Bereich von 250 bis 290 nm über eine kleinere, bevorzugt über eine um mindestens 75 % kleinere und besonders bevorzugt über eine um mindestens 50 % kleinere Absorption verfügt als ein korrespondierendes spritzgegossenes Bauteil, gebildet aus Polystyrol.

7. Spritzgegossenes Bauteil nach einem der vorangehenden Ansprüche,
**gekennzeichnet durch**
eine Fluoreszenz, welche bei Anregung bei einer Wellenlänge von 250 nm und/oder 450 nm und/oder 500 nm vergleichbar ist mit der eines korrespondierenden spritzgegossenen Bauteils, gebildet aus Polystyrol.

8. Spritzgegossenes Bauteil nach einem der vorangehenden Ansprüche, ferner enthaltend
mindestens ein Pigment oder mindestens einen Farbstoff, wobei das mindestens eine Pigment oder der mindestens eine Farbstoff insbesondere pflanzenbasiert und/oder biologisch abbaubar, besonders bevorzugt pflanzenbasiert und biologisch abbaubar ist.

9. Spritzgegossenes Bauteil nach einem der vorangehenden Ansprüche, **dadurch**
**gekennzeichnet, dass**
das Polylactid oder das spritzgegossene Bauteil, insbesondere das Polylactid, über einen Wassergehalt von nicht mehr als 800 ppm, bevorzugt von nicht mehr als 600 ppm und besonders bevorzugt von nicht mehr als 500 ppm verfügt.

10. Spritzgegossenes Bauteil nach einem der vorangehenden Ansprüche, **dadurch**
**gekennzeichnet, dass**
dieses eine Well-Platte (auch Mikrotiterplatte genannt), einen Well-Plattendeckel, Teststäbchen, Teströhrchen, Testkugeln, Petrischalen, einen Kulturmedienbehälter, insbesondere eine Kulturmedienflasche, Pipetten, insbesondere serologische Pipetten, Pipettenbauteile, ein Implantat, eine Handyhülle, ein Behältnis für Lebensmittel oder Kosmetika, Kolben, insbesondere Erlenmeyer-Kolben, Zellkulturgefäße, insbesondere Schüttelkolben wie Schüttelkolben mit Schikanen, T-Flaschen, Reaktionsgefäße, Schraubdeckel, Elektrophoresekammern, Halterungen für Reaktionsgefäße, Probenröhrchen, insbesondere Stuhlprobenröhrchen, Blutprobenröhrchen, Abstrichprobenröhrchen, Halterungen für Probenröhrchen, Urinprobenbecher, Halterungen für Urinprobenbecher, Gehäuse für Antigen-Teststreifen, Lab-on-Chip Systeme, Organ-on-Chip Systeme, Küvetten, Reagenzreservoire, Mikroträger, Haltevorrichtungen zum Transport von medizinischen Behältern, insbesondere Spritzennester, Spritzentuben, Zellzählkammern, Filtergehäuse, Gehäuse für Bioreaktoren, Bioreaktorkessel, Pipettenspitzen-Boxen, Abwurfbehälter, insbesondere für Spritzenkanülen und/oder Glas, Schutzkappen für Spritzenkanülen, Spritzen oder Spritzenstempel darstellt oder umfasst.

11. Verwendung der spritzgegossenen Bauteile nach einem der vorangehenden Ansprüche als Well-Platte, Well-Plattendeckel, Teststäbchen, Teströhrchen, Testkugeln, Petrischalen, Kulturmedienbehälter, insbesondere Kulturmedienflasche, Pipetten, insbesondere serologische Pipetten, Pipettenbauteile, Implantate, Handyhüllen, Behältnis für Lebensmittel oder Kosmetika, Kolben, insbesondere Erlenmeyer-Kolben, Zellkulturgefäße, insbesondere Schüttelkolben wie Schüttelkolben mit Schikanen, T-Flaschen, Reaktionsgefäße, Schraubdeckel, Elektrophoresekammern, Halterungen für Reaktionsgefäße, Probenröhrchen, insbesondere Stuhlprobenröhrchen, Blutprobenröhrchen, Abstrichprobenröhrchen, Halterungen für Probenröhrchen, Urinprobenbecher, Halterungen für Urinprobenbecher, Gehäuse für Antigen-Teststreifen, Lab-on-Chip Systeme, Organ-on-Chip Systeme, Küvetten, Reagenzreservoire, Mikroträger, Haltevorrichtungen zum Transport von medizinischen Behältern, insbesondere Spritzennester, Spritzentuben, Zellzählkammern, Filtergehäuse, Gehäuse für Bioreaktoren, Bioreaktorkessel, Pipettenspitzen-Boxen, Abwurfbehälter, insbesondere für Spritzenkanülen und/oder Glas, Schutzkappen für Spritzenkanülen, Spritzen oder Spritzenstempel.

12. Verfahren zur Herstellung eines spritgegossenen Bauteils nach einem der Ansprüche 1 bis 10, **dadurch gekennzeichnet, dass** man das pflanzenbasierte und/oder, insbesondere und, biologisch abbaubare thermoplastische Polymer, insbesondere Polylactide, in schmelzeflüssiger Form über mindestens zwei, insbesondere mindestens drei, Angusskanäle in die Spritzgießform für das Bauteil einführt.

13. Verfahren nach Anspruch 12, **dadurch gekennzeichnet, dass** man das spritzgegossene Bauteil außerhalb der Spritzgießform abkühlt.

14. Verfahren nach Anspruch 12 oder 13, **dadurch gekennzeichnet, dass** man das pflanzenbasierte und/oder, insbesondere und, biologisch abbaubare thermoplastische Polymer, bevorzugt Polylactide, besonders bevorzugt Polylactide mit einem Wassergehalt von nicht mehr als 800 ppm, bevorzugt von nicht mehr als 600 ppm und besonders bevorzugt von nicht mehr als 500 ppm, in Pulver- oder Granulatform mit dem mindestens einem Monoglycerid einer Fettsäure, insbesondere einer natürlichen Fettsäure, vor dem Aufschmelzen des pflanzenbasierten und/oder, insbesondere und, biologisch abbaubaren thermoplastischen Polymers, insbesondere Polylactide, vermengt.

## Claims

1. Injection-moulded component, comprising or formed from
a plant-based and/or, in particular and, biodegradable thermoplastic polymer, in particular polylactides, and
at least one monoglyceride of a fatty acid, in particular a natural fatty acid, **characterized by**
a content of the plant-based and/or, in particular and, biodegradable thermoplastic polymer, in particular of polylactides, of at least 95.0% by weight, preferably of at least 96.0% by weight and particularly preferably of at least 97.0% by weight, in each case based on the total weight of the component, and
at least two injection points.

2. Injection-moulded component according to Claim 1, **characterized in that** the plant-based and/or, in particular and, biodegradable thermoplastic polymer is selected from the group consisting of polylactides, polyhydroxyalkanoates, poly(butylene succinates) and any desired mixtures thereof, wherein polylactides are preferred.

3. Injection-moulded component according to Claim 1 or 2, **characterized by** at least three injection points.

4. Injection-moulded component according to one of the preceding claims, **characterized by**
a content of the at least one monoglyceride of a fatty acid, in particular of a natural fatty acid, of at most 5.0% by weight, preferably of at most 2.5% by weight and particularly preferably of at most 2.0% by weight, in each case based on the total weight of the component, or **characterized by**
a content of the at least one monoglyceride of a fatty acid, in particular of a natural fatty acid, in the range from 0.5 to 2.5% by weight, preferably in the range from 0.75 to 2.25% by weight and particularly preferably in the range from 1.0 to 2.0% by weight, in each case based on the total weight of the component.

5. Injection-moulded component according to one of the preceding claims, **characterized by**
a content of free fatty acids, in particular natural fatty acids, of less than 0.5% by weight, preferably less than 0.3% by weight and particularly preferably less than 0.1% by weight, in each case based on the total weight of the component, and/or, in particular and,
**characterized by** a content of glycerol of less than 0.3% by weight, preferably less than 0.15% by weight and particularly preferably less than 0.075% by weight, in each case based on the total weight of the component.

6. Injection-moulded component according to one of the preceding claims, **characterized in that**
it has, in the range from 250 to 290 nm, a lower absorption, preferably an absorption which is at least 75% lower and particularly preferably an absorption which is at least 50% lower, than a corresponding injection-moulded component, formed from polystyrene.

7. Injection-moulded component according to one of the preceding claims, **characterized by**
a fluorescence which, when excited at a wavelength of 250 nm and/or 450 nm and/or 500 nm, is comparable with that of a corresponding injection-moulded component, formed from polystyrene.

8. Injection-moulded component according to one of the preceding claims, furthermore comprising
at least one pigment or at least one dye, wherein the at least one pigment or the at least one dye is in particular plant-based and/or biodegradable, particularly preferably plant-based and biodegradable.

9. Injection-moulded component according to one of the preceding claims, **characterized in that**
the polylactide or the injection-moulded component, in particular the polylactide, has a water content of not more than 800 ppm, preferably of not more than 600 ppm and particularly preferably of not more than 500 ppm.

10. Injection-moulded component according to one of the preceding claims, **characterized in that**
it constitutes or comprises a well plate (also called microtiter plate), a well plate cover, test rods, test tubes, test spheres, Petri dishes, a culture medium container, in particular a culture medium bottle, pipettes, in particular serological pipettes, pipette components, an implant, a handy casing, a container for foodstuffs or cosmetics, flasks, in particular Erlenmeyer flasks, cell culture vessels, in particular shaking flasks such as shaking flasks with baffles, T-bottles, reaction vessels, screw covers, electrophoresis chambers, holders for reaction vessels, sample tubes, in particular stool sample tubes, blood sample tubes, swab sample tubes, holders for sample tubes, urine sample cups, holders for urine sample cups, housings for antigen test strips, lab-on-chip systems, organ-on-chip systems, cuvettes, reagent reservoirs, microcarriers, holding devices for transporting medical containers, in particular syringe nests, syringe tubes, cell counting chambers, filter housings, housings for bioreactors, bioreactor vessels, pipette tip boxes, discharge containers, in particular for syringe cannulas and/or glass, protective caps for syringe cannulas, syringes or syringe plungers.

11. Use of the injection-moulded components according to one of the preceding claims as well plate, well plate cover, test rods, test tubes, test spheres, Petri dishes, culture medium containers, in particular culture medium bottle, pipettes, in particular serological pipettes, pipette components, implants, handy casings, container for foodstuffs or cosmetics, flasks, in particular Erlenmeyer flasks, cell culture vessels, in particular shaking flasks such as shaking flasks with baffles, T-bottles, reaction vessels, screw covers, electrophoresis chambers, holders for reaction vessels, sample tubes, in particular stool sample tubes, blood sample tubes, swab sample tubes, holders for sample tubes, urine sample cups, holders for urine sample cups, housings for antigen test strips, lab-on-chip systems, organ-on-chip systems, cuvettes, reagent reservoirs, microcarriers, holding devices for transporting medical containers, in particular syringe nests, syringe tubes, cell counting chambers, filter housings, housings for bioreactors, bioreactor vessels, pipette tip boxes, discharge containers, in particular for syringe cannulas and/or glass, protective caps for syringe cannulas, syringes or syringe plungers.

12. Method for producing an injection-moulded component according to one of Claims 1 to 10, **characterized in that**
the plant-based and/or, in particular and, biodegradable thermoplastic polymer, in particular polylactides, is introduced in molten form via at least two, in particular at least three, sprue channels into the injection mould for the component.

13. Method according to Claim 12, **characterized in that**
the injection-moulded component is cooled outside the injection mould.

14. Method according to Claim 12 or 13, **characterized in that**
the plant-based and/or, in particular and, biodegradable thermoplastic polymer, preferably polylactides, particularly preferably polylactides having a water content of not more than 800 ppm, preferably of not more than 600 ppm and particularly preferably of not more than 500 ppm, is mixed in powder or granular form with the at least one monoglyceride of a fatty acid, in particular a natural fatty acid, before melting of the plant-based and/or, in particular and, biodegradable thermoplastic polymer, in particular polylactides.

## Revendications

1. Pièce moulée par injection, contenant ou constituée d'un polymère thermoplastique à base végétale et/ou, en particulier, biodégradable, en particulier des polylactides, et
au moins un monoglycéride d'un acide gras, en particulier d'un acide gras naturel, **caractérisé par**
une teneur en polymère thermoplastique d'origine végétale et/ou, en particulier, biodégradable, en particulier en polylactides, d'au moins 95,0 % en poids, de préférence d'au moins 96,0 % en poids et de manière particulièrement préférée d'au moins 97,0 % en poids, dans chaque cas par rapport au poids total de la pièce, et
au moins deux points d'injection.

2. Pièce moulée par injection selon la revendication 1, **caractérisé en ce que** le polymère thermoplastique d'origine végétale et/ou, en particulier, biodégradable est choisi dans le groupe constitué par les polylactides, les polyhydroxyalcanoates, les poly(butylènesuccinates) et leurs mélanges quelconques, les polylactides étant préférés.

3. Pièce moulée par injection selon la revendication 1 ou 2, **caractérisé par** au moins trois points d'injection.

4. Pièce moulée par injection selon l'une des revendications précédentes, **caractérisé par**
une teneur en au moins un monoglycéride d'un acide gras, en particulier d'un acide gras naturel, de 5,0 % en poids au maximum, de préférence de 2,5 % en poids au maximum et de préférence encore de 2,0 % en poids au maximum, dans chaque cas par rapport au poids total de la pièce, ou **caractérisé par**
une teneur en au moins un monoglycéride d'un acide gras, en particulier d'un acide gras naturel, comprise entre 0,5 et 2,5 % en poids, de préférence entre 0,75 et 2,25 % en poids et de manière particulièrement préférée entre 1,0 et 2,0 % en poids,
dans chaque cas par rapport au poids total de la pièce.

5. Pièce moulée par injection selon l'une des revendications précédentes, **caractérisé par** une teneur en acides gras libres, en particulier en acides gras naturels,
inférieure à 0,5 % en poids, de préférence inférieure à 0,3 % en poids et de manière particulièrement préférée inférieure à 0,1 % en poids, dans chaque cas par rapport au poids total de la pièce, et/ou, en particulier et,
**caractérisé par** une teneur en glycérine inférieure à 0,3 % en poids, de préférence inférieure à 0,15 % en poids et de manière particulièrement préférée inférieure à 0,075 % en poids, dans chaque cas par rapport au poids total de la pièce.

6. Pièce moulée par injection selon l'une des revendications précédentes, **caractérisé en ce qu'**elle présente, dans la plage de 250 à 290 nm, une absorption inférieure, de préférence inférieure d'au moins 75 % et de préférence encore inférieure d'au moins 50 %, à celle d'une pièce moulé par injection correspondant, formé à partir de polystyrène.

7. Pièce moulée par injection selon l'une des revendications précédentes, **caractérisé par** une fluorescence qui, lorsqu'elle est excité à une longueur d'onde de 250 nm et/ou 450 nm et/ou 500 nm, est comparable à celle d'une pièce moulé par injection correspondant, formé à partir de polystyrène.

8. Pièce moulée par injection selon l'une des revendications précédentes, contenant en outre
au moins un pigment ou au moins un colorant, le au moins un pigment ou le au moins un colorant étant en particulier à base de plantes et/ou biodégradable, de préférence à base de plantes et biodégradable.

9. Pièce moulée par injection selon l'une des revendications précédentes, **caractérisé en ce que**
le polylactide ou le composant moulé par injection, en particulier le polylactide, présente une teneur en eau ne dépassant pas 800 ppm, de préférence ne dépassant pas 600 ppm et de manière particulièrement préférée ne dépassant pas 500 ppm.

10. Pièce moulée par injection selon l'une des revendications précédentes, **caractérisé en ce qu'**elle représente ou comprend une plaque à puits (également appelée plaque de microtitrage), un couvercle de plaque à puits, des bâtonnets de test, des tubes à essai, des billes de test, des boîtes de Pétri, un récipient pour milieu de culture, en particulier une bouteille pour milieu de culture, des pipettes, en particulier des pipettes sérologiques, des composants de pipettes, un implant, une coque de téléphone portable, un récipient pour aliments ou cosmétiques, des flacons, en particulier des flacons Erlenmeyer, des récipients pour culture cellulaire, en particulier des flacons à agiter tels que des flacons à agiter avec chicanes, des flacons en T, des récipients de réaction, des couvercles à vis, des chambres d'électrophorèse, des supports pour récipients de réaction, des tubes à essai, en particulier des tubes à essai pour échantillons de selles, des tubes à essai pour échantillons de sang, tubes à échantillons de frottis, supports pour tubes à échantillons, gobelets à échantillons d'urine, supports pour gobelets à échantillons d'urine, boîtiers pour bandelettes de test antigéniques, systèmes Lab-on-Chip, systèmes Organ-on-Chip, cuvettes, réservoirs de réactifs, micro-supports, dispositifs de fixation pour le transport de récipients médicaux, en particulier les nids à seringues, les tubes à seringues, les chambres de comptage cellulaire, les boîtiers de filtres, les boîtiers pour bioréacteurs, les cuves de bioréacteurs, boîtes pour embouts de pipettes, récipients de collecte, en particulier pour aiguilles de seringues et/ou verre, capuchons de protection pour aiguilles de seringues, seringues ou pistons de seringues.

11. Utilisation des pièce moulés par injection selon l'une des revendications précédentes comme plaque ondulée, couvercle de plaque ondulée, bâtonnets de test, tubes de test, billes de test, boîtes de Pétri, récipients pour milieux de culture, en particulier bouteilles pour milieux de culture, pipettes, en particulier pipettes sérologiques, composants de pipettes, implants, étuis pour téléphones portables, récipients pour aliments ou cosmétiques, ballons, en particulier ballons Erlenmeyer, récipients pour culture cellulaire, en particulier ballons à agiter tels que ballons à agiter avec chicanes, flacons en T, récipients de réaction, couvercles à vis, chambres d'électrophorèse, supports pour récipients de réaction, tubes à essai, en particulier tubes à essai pour échantillons de selles, tubes à essai pour échantillons de sang, tubes à essai pour frottis, supports s pour tubes à essai, flacons pour échantillons d'urine, supports pour flacons pour échantillons d'urine, boîtiers pour bandelettes de test antigéniques, systèmes Lab-on-Chip, systèmes Organ-on-Chip, cuvettes, réservoirs de réactifs, micro-supports, dispositifs de fixation pour le transport de récipients médicaux, en particulier supports pour seringues, tubes pour seringues, chambres de comptage cellulaire, boîtiers de filtres, boîtiers pour bioréacteurs, cuves de bioréacteurs, boîtes pour embouts de pipettes, récipients de collecte, en particulier pour aiguilles de seringues et/ou verre, capuchons de protection pour aiguilles de seringues, seringues ou pistons de seringues.

12. Procédé de fabrication d'un pièce moulé par injection selon l'une des revendications 1 à 10, **caractérisé en ce que**
le polymère thermoplastique d'origine végétale et/ou, en particulier, biodégradable, en particulier les polylactides, est introduit sous forme liquide fondue dans le moule d'injection pour le composant via au moins deux, en particulier au moins trois, canaux d'injection.

13. Procédé selon la revendication 12, **caractérisé en ce que**
la pièce moulé par injection est refroidi à l'extérieur du moule d'injection.

14. Procédé selon la revendication 12 ou 13, **caractérisé en ce que**
le polymère thermoplastique à base végétale et/ou, en particulier, biodégradable, de préférence des polylactides, de manière particulièrement préférée des polylactides ayant une teneur en eau ne dépassant pas 800 ppm, de préférence ne dépassant pas 600 ppm et de manière particulièrement préférée ne dépassant pas 500 ppm, sous forme de poudre ou de granulés, avec au moins un monoglycéride d'un acide gras, en particulier d'un acide gras naturel, avant la fusion du polymère thermoplastique d'origine végétale et/ou, en particulier, biodégradable, en particulier des polylactides.
